(19) Europäisches Patentamt
European Patent Office
Office européen des brevets

(11) Publication number: **0 123 520**
**B1**

(12) # EUROPEAN PATENT SPECIFICATION

(45) Date of publication of patent specification: **17.08.88**

(51) Int. Cl.$^4$: **C 07 D 417/12**

(21) Application number: **84302666.7**

(22) Date of filing: **19.04.84**

(54) **Process for preparing base salts of piroxicam deposited on a pharmaceutically-acceptable carrier.**

(30) Priority: **25.04.83 US 488303**

(43) Date of publication of application:
**31.10.84 Bulletin 84/44**

(45) Publication of the grant of the patent:
**17.08.88 Bulletin 88/33**

(84) Designated Contracting States:
**AT BE CH DE FR GB IT LI LU NL SE**

(56) References cited:
**EP-A-0 066 459**

**CHEMICAL ABSTRACTS, vol. 99, no. 21, 21st November 1983, page 572, no. 175210q, Columbus, Ohio, US; & ES - A - 512 563 (FORDONAL S.A.) 16-02-1983**

**CHEMICAL ABSTRACTS, vol. 99, no. 26, 26th December 1983, page 383, no. 218582g, Columbus, Ohio, US; & JP - A - 58 118 584 (CIBA-GEIGY A.-G.) 14-07-1983**

(73) Proprietor: **PFIZER INC.**
**235 East 42nd Street**
**New York, N.Y. 10017 (US)**

(72) Inventor: **Noseworthy, Melvin MacKenzie**
**263 Whalehead Road Gales Ferry**
**New London County Connecticut (US)**

(74) Representative: **Wood, David John et al**
**Pfizer Limited Ramsgate Road**
**Sandwich Kent CT13 9NJ (GB)**

**EP 0 123 520 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Piroxicam, which is identified chemically as 4-hydroxy-2-methyl-*N*-(2-pyridyl)-2H-1,2-benzothiazine-3-carboxamide 1,1-dioxide, is a non-steroidal anti-inflammatory agent which is finding increasing clinical use in the treatment of rheumatoid arthritis in man. For this purpose it is usually administered orally, in the form of capsules containing the anhydrous compound (which has a low solubility in water) and a pharmaceutically-acceptable carrier. However, piroxicam also has analgesic properties, and for this indication it is preferable to administer piroxicam as a water-soluble salt, to facilitate rapid biological absorption.

Accordingly, it is an object of this invention to provide a process for preparing certain water-soluble, base salts of piroxicam. More particularly, this invention provides a process for preparing certain water-soluble, base salts of piroxicam, in which the salt has been deposited on a pharmaceutically-acceptable carrier. The compositions so obtained can be incorporated into dosage forms which have a rapid rate of disintegration, a rapid rate of dissolution and a rapid rate of absorption after oral administration to a mammalian subject.

Utilization of the process of this invention offers advantages over conventional methods of preparing dosage forms of a salt of piroxicam in that it avoids the necessity for isolating, purifying and characterizing the particular salt, and also the mechanical losses and costs attendant upon isolating a particular salt.

Conventional methods for preparing salts of piroxicam are taught in United States patent No. 3,591,584 and published European patent application No. 66459 (1982).

This invention provides a process for preparing a base salt of piroxicam deposited on a pharmaceutically-acceptable solid carrier, said base salt of piroxicam being selected from the group consisting of the sodium salt, the ethanolamine salt and the diethanolamine salt, which comprises:

(a) combining piroxicam, a base reagent selected from sodium hydroxide, trisodium phosphate, ethanolamine and diethanolamine and a pharmaceutically-acceptable solid carrier, in a solvent selected from water, alkanols having one to four carbons and mixtures thereof, at a temperature in the range from 10 to 40°C.; wherein the piroxicam and the basic reagent are contacted in substantially equimolar proportions, the ratio of the carrier to the piroxicam is in the range from 5:1 to 30:1 by weight and the ratio of the solvent to the carrier is in the range from 1:1 to 1:5 by weight; with the proviso that when said basic reagent is trisodium phosphate, said solvent must be at least 90% water by weight;

(b) granulating the product of step (a); and

(c) removing the solvent from the product of step (b).

Typical pharmaceutically-acceptable solid carriers which can be used are microcrystalline cellulose and lactose, optionally in admixture with sodium citrate.

In carrying out the process of this invention, the preferred ratio of the carrier to the piroxicam is in the range from 10:1 to 25:1, and the preferred ratio of the solvent to the carrier is in the range from 1:1.2 to 1:3.

Piroxicam as used in the process of this invention is the compound of the formula

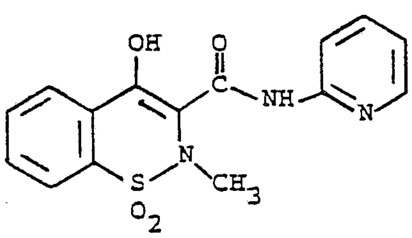

and when reference is made to piroxicam in step (a) of the process of this invention, said reference is intended to refer both to anhydrous piroxicam and also the various non-toxic solvates thereof.

Step (a) of the process of this invention involves combining piroxicam, a base, a solid carrier and a solvent and the manner and order in which they are combined is not critical. For example, the piroxicam can be blended with the carrier, and then this blend can be added with stirring to a solution of the base in the solvent. Alternatively, a solution of the piroxicam salt can be prepared by adding both piroxicam and the base to the solvent, and then the piroxicam salt solution can be used to wet the carrier. In yet another variation, which can be used especially when a liquid base is being used, the piroxicam and the carrier are first blended together, then the liquid base is added, and finally the mixture so obtained is wetted with the solvent.

The solvent which is used in step (a) of the process of this invention is selected from water and alkanols having one to four carbon atoms, and mixtures thereof. However, when using mixtures, it is advantageous to use a homogeneous mixture. Moreover, when trisodium phosphate is used as the basic reagent, the solvent system for step (a) must be at least 90% water by weight.

During the process of step (a) of this invention, various conventional mixing, agitating and blending techniques are commonly used.

In regard to the pharmaceutically-acceptable carrier to be employed in step (a) of the process of this invention, a variety of such agents can be employed. The major requirements of the pharmaceutically-acceptable carrier are that it is a solid, it does not adversely interact with either piroxicam or the solvent used in step (a) of this process, and it is non-toxic to the mammalian host which is to be treated ultimately. Typical carriers which are used are microcrystalline cellulose, lactose, calcium sulfate and dicalcium phosphate, and mixtures thereof. Particularly preferred is microcrystalline cellulose, especially the food grade, microcrystalline, partially depolymerized cellulose obtained by treating plant cellulose with mineral acid as described in United States patent No. 2,978,446. If desired, certain buffers such as sodium citrate can form part of the pharmaceutically-acceptable carrier. The buffer will normally be present in an amount, by weight, which is equal to or less than the other ingredients. Also, if desired, small amounts, e.g. 10% or less by weight of the total carrier used, of other ingredients such as dispersing agents (e.g. starch and alginic acid), binding agents (e.g. polyvinylpyrrolidone, sucrose, gelatin and acacia) and lubricating agents (e.g. talc, magnesium stearate and sodium lauryl sulfate) may be present.

In step (a) of the process of this invention, the piroxicam and the basic reagent are normally contacted in equimolar proportions. However, a slight excess (e.g. 5 molar percent) of either component can be used. Larger excesses of either compound are not desirable.

As far as the proportion of piroxicam and the pharmaceutically-acceptable carrier in step (a) are concerned, the ratio of the carrier to the piroxicam is in the range from 5:1 to 30:1, and preferably 10:1 to 25:1, by weight. In like manner, the ratio of the solvent to the carrier is in the range from 1:1 to 1:5, and preferably 1:1.2 to 1:3, by weight.

Step (b) of the process of this invention involves granulating the product of step (a). The term "granulating", as used herein, has the normal, conventional meaning commonly used in standard pharmaceutical practice. Essentially, it refers to a process in which the individual particles of the material produced in step (a) are accreted to form agglomerates. This process converts the small particles in the material produced in step (a) into larger particles, the agglomerates, which are denser, more spherical in shape and generally more uniform in size. The granulation is carried out in conventional fashion, and basically it involves blending or kneading the product of step (a) until the desired amount of accretion has taken place. In the product of step (b), the particle size will normally be in the range from 125 to 400 micrometers.

Step (c) of the process of this invention involves removal of the solvent that was added in step (a). This is carried out in conventional fashion. It can be carried out simply by exposing the product of step (b) to air, or an inert gas such as nitrogen or argon, at relative humidity of less than 50%, at a tempereature in the range from 20 to 80°C., and a pressure in the range from 0.1 mm of mercury to atmospheric pressure. However, the preferred drying temperature is about 50°C., and it is usually convenient to carry out the drying at about atmospheric pressure. Under these latter conditions, drying times of a few hours, e.g. 2 to 20 hours are commonly used. In any event, drying is stopped when the solvent concentration in the product has been reduced to about 0.1% or less.

The compositions produced by the process of this invention can be used directly as a medicinal agent. However, more usually, they are converted into tablets suitable for oral administration to a human subject. For the latter purpose, the compositions are usually combined with further amounts of a pharmaceutically-acceptable carrier, e.g. microcrystalline cellulose, lactose or dicalcium phosphate. Various disintegrants such as starch, e.g. potato starch or tapioca starch, and binding agents e.g. polyvinylpyrrolidone, sucrose, gelatin or acacia, can be added. Also, lubricating agents, such as magnesium stearate and/or sodium lauryl sulfate, are commonly added. After blending, the final composition is compressed into tablets in conventional fashion.

Tablets so produced will normally contain a piroxicam salt in an amount equivalent to from 5 to 30 mg of piroxicam free acid, and they will normally be administered orally to human subjects once a day for the control of rheumatoid arthritis. However, as indicated hereinbefore, these tablets are particularly suitable for analgesic use, on account of their rapid disintegration, dissolution and absorption after oral administration to a human subject.

The following Examples are being provided solely for the purpose of further illustration.

## Example 1

### Sodium Salt of Piroxicam Deposited on Microcrystalline Cellulose

To a blended mixture of 10.0 g of piroxicam and 100.0 g of Avicel® pH 101 (a microcrystalline cellulose) was added, with stirring, a solution of 1.20 g of sodium hydroxide in 80 g of ethanol. The resulting mixture was granulated and then the solvent was removed by heating at 50°C. for 16 hours at atmospheric pressure. This afforded the title composition.

## Example 2

### Sodium Piroxicam Tablets

The product of Example 1 was passed through a 60-mesh sieve, blended and then it was combined with 38.0 g of Avicel® pH 101 and 58.72 g of Starch 1500 (a pregelatinized corn starch). The product was blended, passed through a 40-mesh sieve and reblended. To the latter product was added 2.08 g of a 9:1 mixture (by weight) of magnesium stearate and sodium lauryl sulfate, and the mixture was blended for five minutes. The resulting mixture was converted into 2,000 tablets on a conventional tabletting machine.

Example 3

Sodium Salt of Piroxicam Deposited on Microcrystalline Cellulose and Sodium Citrate

A blended mixture of 5.0 g of piroxicam, 50.0 g of microcrystalline cellulose and 50 g of sodium citrate was passed through a 40-mesh sieve. To the resulting powder was added a solution of 0.60 g of sodium hydroxide in 35 g of ethanol. The resulting mixture was granulated and then the product was dried at 50°C. for 4 hours at atmospheric pressure. This afforded the title composition.

Example 4

Sodium Piroxicam Tablets

The product of Example 3 was combined with 6.0 g of Explotabs® (a cross-linked sodium carboxymethyl cellulose) and 37 g of Avicel® pH 101 and the mixture was blended. Then, 1.40 g of a 9:1 mixture (by weight) of magnesium stearate and sodium lauryl sulfate was added and the mixture was again blended. This final mixture was converted into 1,000 tablets on a conventional tabletting machine.

Example 5

Monoethanolamine Salt of Piroxicam Deposited on Microcrystalline Cellulose

To a blended mixture of 5.0 g of piroxicam and 69.0 g of Avicel® pH 103 (a microcrystalline cellulose) was added a solution of 0.92 g of monoethanolamine in 40.0 g of ethanol. The resulting mixture was granulated, and then the solvent was removed from the wet blend by heating at 50°C. for 5 hours at atmospheric pressure under nitrogen. This afforded the title composition.

The diethanolamine salt of piroxicam can be deposited onto microcrystalline cellulose by repeating the above procedure using diethanolamine in place of monoethanolamine.

Example 6

Monoethanolamine Piroxicam Tablets

A mixture of the product of Example 5 and 29.04 g of Starch 1500 (a pregelatinized corn starch) was blended, then it was passed through a 60-mesh sieve three times and reblended. To this latter blend was added 1.04 g of a 9:1 mixture (by weight) of magnesium stearate and sodium lauryl sulfate and the mixture was blended for 5 minutes. The resulting blend was converted into 1,000 tablets on a conventional tabletting machine.

Example 7

Sodium Salt of Piroxicam Deposited on Lactose and Sodium Citrate

To a solution of 0.6 g of sodium hydroxide in ethanol was added 5.0 g of piroxicam, to give a clear, light yellow solution. This solution was added to a blended mixture of 79.0 g of lactose and 50.0 g of anhydrous sodium citrate. The resulting mixture was granulated and then the solvent was removed by evaporation at 50°C. for four hours. This afforded the title composition.

Example 8

Sodium Salt of Piroxicam Deposited on Microcrystalline Cellulose

To a solution of 5.73 g of trisodium phosphate dodecahydrate in 100 g of water was added 5.00 g of piroxicam. The mixture was heated to 65°C. to obtain a clear solution, and then it was cooled back to room temperature. To the cooled mixture was added 75 g of Avicel® pH 101 (a microcrystalline cellulose) and the resulting mixture was granulated. The solvent was removed by heating at 50°C. for 16 hours. This afforded the title composition.

**Claims**

1. A process for preparing a base salt of piroxicam deposited on a pharmaceutically-acceptable solid carrier, said base salt of piroxicam being the sodium salt, the ethanolamine salt or the diethanolamine salt, characterized by:

(a) combining piroxicam, a basic reagent selected from sodium hydroxide, trisodium phosphate, ethanolamine and diethanolamine and a pharmaceutically-acceptable solid carrier, in a solvent selected from water, alkanols having one to four carbons and mixtures thereof, at a temperature in the range from 10 to 40°C;

wherein the piroxicam and the basic reagent are contacted in substantially equimolar proportions, the ratio of the carrier to the piroxicam is in the range from 5:1 to 30:1 by weight and the ratio of the solvent to the carrier is in the range from 1:1 to 1:5 by weight;

with the proviso that when said basic reagent is trisodium phosphate, said solvent must be at least 90% water by weight;

(b) granulating the product of step (a); and

(c) removing the solvent from the product of step (b).

2. A process according to claim 1, charcterized in that step (a) is carried out by combining a solution of the basic agent in the solvent with a mixture of piroxicam and the carrier.

3. A process according to claim 1, characterized in that step (a) is carried out by combining a solution of piroxicam and the basic agent in the solvent with the carrier.

4. A process according to any of claims 1 to 3, characterized in that the basic reagent is sodium hydroxide or ethanolamine.

5. A process according to any one of claims 1 to 4, characterized in that the carrier is substantially microcrystalline cellulose, a mixture of microcrystalline cellulose and sodium citrate, lactose, or a mixture of lactose and sodium citrate.

6. A process according to any one of claims 1 to 5, characterized in that the solvent is ethanol.

7. A process according to any one of claims 1 to 6, characterized in that the ratio of the carrier to the piroxicam is in the range from 10:1 to 25:1 by weight and the ratio of the solvent to the carrier is in the range from 1:1.2 to 1:3 by weight.

**Patentansprüche**

1. Verfahren zur Herstellung eines Basensalzes von Piroxicam, abgeschieden auf einem pharmazeutisch annehmbaren festen Träger, wobei das Basensalz von Piroxicam das Natrium-, Ethanolamin- oder Diethanolaminsalz ist, gekennzeichnet durch

(a) Zusammenbringen von Piroxicam, einem basischen Reagens, ausgewählt unter Natriumhydroxid, Trinatriumphosphat, Ethanolamin und Diethanolamin, und einem pharmazeutisch annehmbaren festen Träger in einem Lösungsmittel, ausgewählt unter Wasser, Alkanolen mit 1 bis 4 Kohlenstoffatomen und deren Gemischen, bei einer Temperatur im Bereich von 10 bis 40°C, wobei das Piroxicam und das basische Reagens in praktisch äquimolaren Mengen zusammengebracht werden, das Verhältnis von Träger zu Piroxicam im Bereich von 5:1 bis 30:1 auf Gewichtsbasis und das Verhältnis von Lösungsmittel zu Träger im Bereich von 1:1 bis 1:5 auf Gewichtsbasis liegt, mit der Maßgabe, daß, wenn das basische Reagens Trinatriumphosphat ist, das Lösungsmittel zu wenigstens 90 Gew.-% Wasser sein muß,

(b) Granulieren des Prudukts der Stufe (a) und

(c) Entfernen des Lösungsmittels aus dem Produkt der Stufe (b).

2. Verfahren nach Anspruch 1, dadurch gekennzeichnet, daß Stufe (a) durch Zusammenbringen einer Lösung des basischen Mittels im Lösungsmittel mit einem Gemisch aus Piroxicam und dem Träger durchgeführt wird.

3. Verfahren nach Anspruch 1, dadurch gekennzeichnet, daß Stufe (a) durch Zusammenbringen einer Lösung aus Piroxicam und dem basischen Mittel im Lösungsmittel mit dem Träger erfolgt.

4. Verfahren nach irgendeinem der Ansprüche 1 bis 3, dadurch gekennzeichnet, daß das basische Reagens Natrium-hydroxid oder Ethanolamin ist.

5. Verfahren nach irgendeinem der Ansprüche 1 bis 4, dadurch gekennzeichnet, daß der Träger praktisch mikrokristalline Cellulose, ein Gemisch aus mikrokristalliner Cellulose und Natriumcitrat, Lactose oder ein Gemisch aus Lactose und Natriumcitrat ist.

6. Verfahren nach irgendeinem der Ansprüche 1 bis 5, dadurch gekennzeichnet, daß das Lösungsmittel Ethanol ist.

7. Verfahren nach irgendeinem der Ansprüche 1 bis 6, dadurch gekennzeichnet, daß das Verhältnis von Träger zu Piroxicam im Bereich von 10:1 bis 25:1 auf Gewichtsbasis und das Verhältnis von Lösungsmittel zu Träger im Bereich von 1:1,2 bis 1:3 auf Gewichtsbasis liegt.

**Revendications**

1. Procédé de préparation d'un sel basique de piroxicam déposé sur un support solide pharmaceutiquement acceptable, ledit sel basique de piroxicam étant le sel de sodium, le sel d'éthanolamine ou le sel de diéthanolamine, caractérisé en ce qu'il consiste:

(a) à mélanger du piroxicam, un réactif basique choisi entre l'hydroxyde de sodium, le phosphate trisodique, l'éthanolamine et la diéthanolamine, et un support solide pharmaceutiquement acceptable dans un solvant choisi entre l'eau, des alcanols ayant un à quatre atomes de carbone et leurs mélanges, à une température comprise dans l'intervalle de 10 à 40°C;

le piroxicam et le réactif basique étant mis en contact en des proportions pratiquement équimolaires, le rapport du support au piroxicam étant compris dans l'intervalle de 5:1 à 30:1 en poids et le rapport du solvant au support étant compris dans l'intervalle de 1:1 à 1:5 en poids;

sous réserve que, lorsque ledit réactif basique est le phosphate trisodique, ledit solvant renferme au moins 90% en poids d'eau;

(b) à granuler le produit de l'étape (a) et

(c) à éliminer le solvant du produit de l'étape (b).

2. Procédé suivant la revendication 1, caractérisé en ce que l'étape (a) est mise en oeuvre en mélangeant une solution de l'agent basique dans le solvant à un mélange de piroxicam et du support.

3. Procédé suivant la revendication 1, caractérisé en ce que l'étape (a) est mise en oeuvre en mélangeant au support une solution de piroxicam et de l'agent basique dans le solvant.

4. procédé suivant l'une quelconque des revendications 1 à 3, caractérisé en ce que le réactif basique est l'hydroxyde de sodium ou l'éthanolamine.

5. Procédé suivant l'une quelconque des revendications 1 à 4, caractérisé en ce que le support est de la

cellulose fortement microcrystalline, un mélange de cellulose microcristalline et de citrate de sodium, le lactose, ou un mélange de lactose et de citrate de sodium.

6. Procédé suivant l'une quelconque des revendications 1 à 5, caractérisé en ce que le solvant est l'éthanol.

7. Procédé suivant l'une quelconque des revendications 1 à 6, caractérisé en ce que le rapport du support au piroxicam est compris dans l'intervalle de 10:1 à 25:1 en poids, et le rapport du solvant au support est compris dans l'intervalle de 1:1,2 à 1:3 en poids.